Europäisches Patentamt

European Patent Office

Office européen des brevets

(19)

(11) Publication number: **0 029 281**
**B1**

## (12) EUROPEAN PATENT SPECIFICATION

(45) Date of publication of patent specification: **11.07.84**

(51) Int. Cl.³: **A 61 B 1/00, A 61 B 1/12**

(21) Application number: **80302590.7**

(22) Date of filing: **30.07.80**

(54) Scirroscope.

(30) Priority: **20.11.79 JP 150521/79**

(43) Date of publication of application:
**27.05.81 Bulletin 81/21**

(45) Publication of the grant of the patent:
**11.07.84 Bulletin 84/28**

(84) Designated Contracting States:
**AT BE CH DE FR GB IT LI NL SE**

(56) References cited:
**DE - A - 2 528 543**
**FR - A - 1 347 596**
**FR - A - 1 562 267**
**FR - A - 2 397 930**
**US - A - 3 010 357**
**US - A - 3 091 235**

(73) Proprietor: **OLYMPUS OPTICAL CO., LTD.**
**43-2, 2-chome, Hatagaya Shibuya-ku**
**Tokyo 151 (JP)**

(72) Inventor: **Takagi, Takeji**
**3092-1, Aihara-Machi Machida-Shi**
**Tokyo (JP)**

(74) Representative: **Jones, Colin et al,**
**W.P. THOMPSON & CO. Coopers Building Church**
**Street**
**Liverpool L1 3AB (GB)**

Courier Press, Leamington Spa, England.

## Description

The invention relates to a scirroscope comprising a straight rigid sheath and internal parts housed in said sheath and bonded in place therein, which parts include an observation optical system and an illumination optical system. Such a scirroscope is already known from FR—A—1562267.

A scirroscope has a disadvantage in that it is susceptible to shocks because of the principal materials, such as metal or glass, which are used to form it. In particular, if the scirroscope is dropped inadvertently during use, it is likely that the shocks applied will cause a misalignment of the optical system or a water leakage. A scirroscope includes tubes and other parts which are directly joined to each other by adhesives. These parts exhibit different coefficients of thermal expansion, which may give rise to exfoliation if the scirroscope is sterilized under heat or cleaned with hot water.

It is an object of the invention to eliminate above disadvantages, by providing a scirroscope which is of more robust construction and less prone to experiencing extreme temperature gradients during sterilization.

From FR—A—1347596 it is known to cover internal segments of a flexible endoscope with a resilient envelope for the purpose of holding the internal segments in assembled relationship without significantly impairing flexibility. However, the segments are not bonded to such resilient member and a flexible endoscope is not in any event susceptible to damage to the same extent as a rigid scirroscope.

The present invention provides a scirroscope comprising a straight rigid sheath and internal parts housed in said sheath and bonded in place therein, which parts include an observation optical system and an illumination optical system, characterised in that the interior of said sheath or the exterior of at least one of said internal parts or both such interior and exterior(s) is or are coated with an elastic resilient, shock-absorbing member which is bonded both to the sheath interior or part exterior, as the case may be, and to the adjoining internal part(s) or adjoining sheath portion, respectively.

The provision of a coating of an elastic or resilient material, such as rubber or plastics, on the internal or external periphery of a sheath or shell tube, optics tube, channel and optical fibre bundle, which form a portion of a scirroscope adapted to be inserted into a coeliac cavity and which are of materials such as glass, metal and glass fibre, is effective to attenuate any external shock, which may be produced as a result of the scirroscope being dropped during use, as it is transmitted to an inner part or parts, thus avoiding a misalignment of the optical system or breakage of lenses. The elastic material such as rubber or plastics exhibits a lower thermal conductivity, minimizing the conduction of heat to the interior if heat is applied to the scirroscope when sterilizing it in an autoclave or other equipment. The resilience of the elastic material accommodate for some differences in the coefficient of thermal expansion between the parts, thus acting as a buffering element therebetween to eliminate the occurrence of exfoliation or cracks.

The invention is further described, by way of example, with reference to the drawings, in which:—

Fig. 1 is a side elevation of an example of a conventional scirroscope, with a portion to be inserted into a coeliac cavity shown in section; and

Figs. 2 to 4, (a) and (b) are respective axial and transverse sections of several embodiments of the invention, respectively.

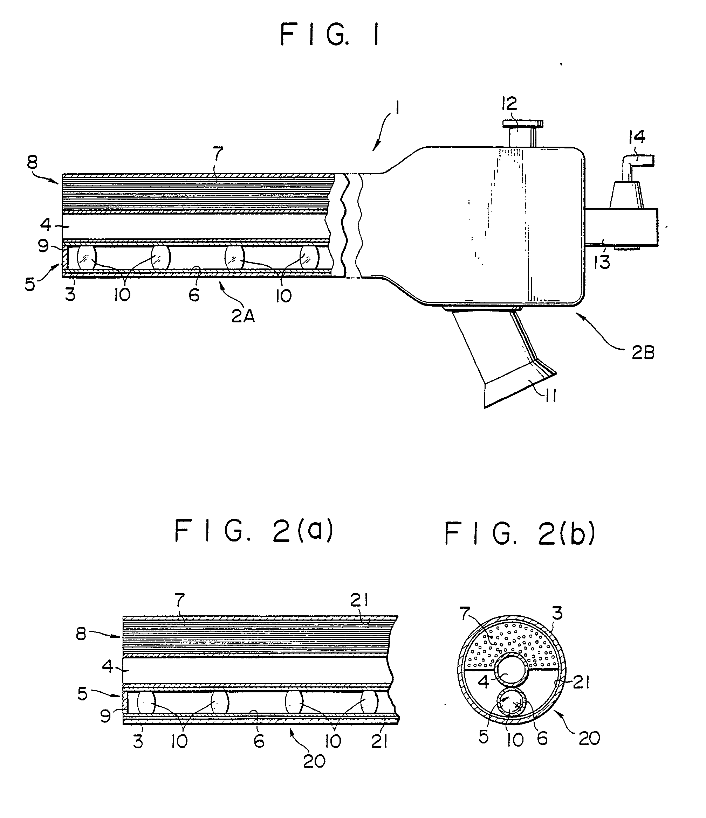

Fig. 1 illustrates a conventional scirroscope 1, including a portion 2A adapted to be inserted into a coeliac cavity and an operating end 2B. The portion 2A comprises a shell tube or sheath 3 formed of a metal and configured as a pipe in which are disposed a channel 4 for feeding water or passing a treatment instrument or the like, and an optical tube 6 having an observation optical system 5 disposed therein as well as an illumination optical system 8, formed by a bundle of optical fibres 7. The observation system 5 includes a protective glass 9 attached to its distal end, and a plurality of lenses 10 which are disposed in the tube 6 and are used to project an image being observed. The operating end 2B comprises an observation eyepiece assembly 11, a fixture 12 for an illumination light guide, and a fitting 13 for connection with a hose which is used to feed water into the channel 4 and having a feed-water control cock 14 associated therewith.

Parts of the insertion portion 2A are formed of metal, glass, glass fibre and like materials which are susceptible to damage by shock. Additionally, these parts are directly joined to each other by adhesives, and this causes any externally applied shock to be directly transmitted to objects located within them, with consequences that the optical axis of the optical system may be misaligned and the lenses broken. Also, a relative displacement between the parts as well as cracks in them are caused by different coefficients of thermal expansion thereof.

Figs. 2(a) and (b) are sections of essential parts of an insertion portion 20 of a scirroscope according to one embodiment of the invention which is adapted to be inserted into a coeliac cavity. Corresponding parts to those shown in Fig. 1 are designated by like reference characters, and hence will not be further described.

An elastic member 21 is integrally bonded, as by adhesive or crimping, to the internal periphery of the sheath 3 of the insertion portion 20, and an adhesive is applied to the interface between the internal periphery of the elastic member and another member which contacts it, such as the optics tube 6 and the fibre bundle 7. In this manner, such inner member is integrally bonded to the elastic member 21 as by adhesion or crimping.

The presence of the elastic member 21, which absorbs some of the shock applied to the sheath 3, as might be produced if the latter is dropped, minimizes the influence of the shock upon internal parts, thus minimizing the likelihood that the optical system 5 may be damaged. The reduced thermal conductivity of the elastic member 21, formed by rubber, plastics or the like, retards the heat transfer to the interior, whereby a temperature gradient which causes an exfoliation or cracks is advantageously reduced.

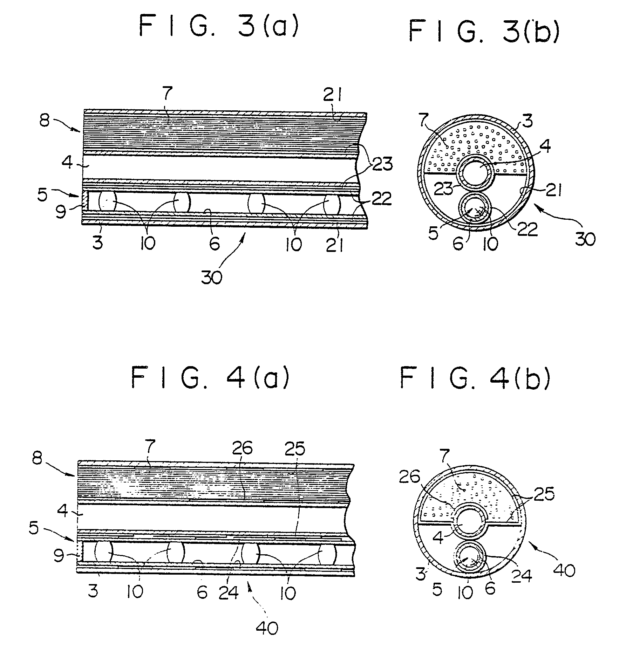

Figs. 3(a) and (b) show another embodiment of the invention where an insertion portion 30 of scirroscope shown is provided with a coating in the form of an elastic member applied to the inner periphery of the sheath 3, in the same manner as shown in Fig. 2. Additionally, it is provided with elastic members 22 and 23 on the outer periphery of the optics tube 6 and the channel 4, respectively. Other parts correspond to those shown in Figs. 1 and 2.

Since the insertion portion 30 is provided with elastic members 21, 22, 23, formed of rubber, plastics or the like, which coat the internal periphery of the sheath 3 and the external periphery of the optics tube 6 and the channel 4, the insertion portion 30 exhibits a heat and shock resistance which are comparable to or even exceed those of the insertion portion 20 shown in Fig. 2.

Figs. 4(a) and (b) show a further embodiment of the invention where an insertion portion 40 of scirroscope which is adapted to be inserted into a coeliac cavity is provided with a plurality of ring-shaped elastic members 24 coating the external periphery of the optics tube 6, a plurality of tubular elastic members 25 of a reduced length coating the external periphery of both the fibre bundle 7 and the channel 4, and an elastic member 26 in a trapezoidal form and having a reduced length covering one-half the external periphery of the channel 4. Again, corresponding parts to those shown in Figs. 2 and 3 are designated by like reference characters and will not be described.

The use of several elastic members 24, 25, 26, though of reduced lengths, to cover various parts in the embodiment of Fig. 4 is also effective to absorb shocks applied, preventing the occurrence of exfoliation or cracks under heat.

## Claims

1. A scirroscope comprising a straight rigid sheath (3) and internal parts (4, 5, 7) housed in said sheath and bonded in place therein, which parts include an observation optical system (5) and an illumination optical system (7), characterised in that the interior of said sheath (3) or the exterior of at least one of said internal parts (4, 5, 7) or both such interior and exterior is or are coated with an elastic, resilient, shock absorbing member (21—26) which is bonded both to the sheath interior or part exterior, as the case may be and to the adjoining internal parts or adjoining sheath portion, respectively.

2. A scirroscope according to claim 1, in which the internally housed parts include a channel (4) which is used to feed water or to pass a treatment instrument, the observation system (5) includes an optical tube (6) in which the optical elements of the observation system (5) are disposed and the illumination system comprises a bundle (7) of optical fibres.

3. A scirroscope according to claim 1 or 2, in which the resilient member (21—26) coats the internally housed part (4, 6, 7) either partly or entirely.

4. A scirroscope according to claim 1, 2 or 3, in which the resilient member (21—26) is formed of rubber or plastics.

## Patentansprüche

1. Endoskop mit einer geraden starren Hülle (3) und in der Hülle angeordneten und darin an ihrer Stelle festgelegten inneren Teilen (4, 5, 7), die ein optisches Beobachtungssystem (5) und ein optisches Beleuchtungssystem (7) enthalten, dadurch gekennzeichnet, daß das Innere der Hülle (3) oder das Äußere mindestens eines der inneren Teile (4, 5, 7) oder sowohl das Innere als auch das Äußere mit einem elastischen, nachgiebigen, shockabsorbierenden Element (21—26) überzogen ist, das sowohl mit dem Hülleninneren oder dem Teileäußeren, je nach Lage der Sache, als auch mit den benachbarten inneren Teilen bzw. dem benachbarten Hüllenabschnitt verbunden ist.

2. Endoskop nach Anspruch 1, bei dem die intern angeordneten Teile einen Kanal (4) enthalten, der zur Zufuhr von Wasser oder zum Durchlassen eines Behandlungsinstrumentes verwendet wird, das Beobachtungssystem (5) ein optisches Rohr (6) enthält, in dem die optischen Elemente des Beobachtungssystems (5) angeordnet sind, und das Beleuchtungssystem ein Bündel (7) optischer Fasern aufweist.

3. Endoskop nach Anspruch 1 oder 2, bei dem das nachgiebige Element (21—26) das intern angeordnete Teil (4, 6, 7) entweder teilweise oder vollständig bedeckt.

4. Endoskop nach Anspruch 1, 2 oder 3, bei dem das nachgiebige Element (21—26) aus Gummi oder Kunststoff gebildet ist.

## Revendications

1. Un endoscope comprenant un manchon rigide droit (3) dans lequel sont logées des parties internes (4, 5, 7) qui y sont fixées en place et qui comportent un système optique d'observation (5) et un système optique d'éclairage (7), caractérisé en ce que l'intérieur dudit manchon (3) ou l'extérieur d'au moins une desdites parties internes (4, 5, 7) ou bien ledit intérieur et ledit extérieur est ou sont revêtus d'un organe amortisseur de choc élastique (21—26) lié tant à l'intérieur de manchon ou à l'extérieur de partie selon le case qu'aux parties internes avoisinantes ou portion de manchon avoisinante respectivement.

2. Un endoscope selon la revendication 1, dans lequel les parties logées intérieurement comprennent un canal (4) qui sert à alimenter en eau ou à faire passer un instrument de traitement, le système d'observation (5) comprend un tube optique (6) dans lequel sont disposés des éléments optiques du système d'observation (5) et le système d'éclairage comprend un faisceau (7) de fibres optiques.

3. Un endoscope selon la revendication 1 ou 2, dans lequel l'organe élastique (21—26) revêt la partie logée intérieurement (4, 6, 7) soit partiellement soit entièrement.

4. Endoscope selon la revendication 1, 2 ou 3, dans lequel l'organe élastique (21—26) est formé de caoutchouc ou matière plastique.

# F I G. I

# F I G. 2(a)

# F I G. 2(b)

1

F I G. 3(a)

F I G. 3(b)

F I G. 4(a)

F I G. 4(b)